# EUROPEAN PATENT SPECIFICATION

(11) **EP 1 789 408 B1**
(45) Date of publication and mention of the grant of the patent: **16.04.2008**
(21) Application number: 05779469.5
(22) Date of filing: 22.08.2005
(51) Int. Cl.: C07D 307/32, A61P 5/28, A61K 31/341

(54) **ANDROGEN MODULATORS**
ANDROGENMODULATOREN
MODULATEURS ANDROGENES

(30) Priority: 31.08.2004 US 605896 P
(43) Date of publication of application: 30.05.2007
(73) Proprietor: Warner-Lambert Company LLC, New York, NY 10017 (US)
(72) Inventor: BARRETT, Stephen Douglas, Pfizer Global R & D, Ann Arbor, MI 48105 (US); DU, Daniel Yunlong, Pfizer Global R & D, Ann Arbor, MI 48105 (US); HU, Lain-Yen, Pfizer Global R & D, Ann Arbor, MI48105 (US); LEFKER, Bruce Allen, Pfizer Global R & D, Groton, CT 06340 (US); RAHEJA, Raj Kumar, Pfizer Global R & D, Ann Arbor, MI 48105 (US); SEXTON, Karen Elaine, Pfizer Global R & D, Ann Arbor, MI 48105 (US); SMITH, Yvonne Dorothy, Pfizer Global R & D, Ann Arbor, MI 48105 (US)
(74) Representative: Pringot, Thomas
(86) International application number: PCT/IB2005/002700
(87) International publication number: WO 2006/024942

(56) References cited:
- WO-A-20/05049574
- WO-A-20/05080320
- WO-A-20/05100305
- WO-A-20/05102990
- DE-A1- 10 218 963

## Description

### FIELD OF THE INVENTION

The present invention is directed to a new class of 4-cyctoalkoxy benzonitriles and to their use as androgen receptor modulators. Other aspects of the invention are directed to the use of these compounds to decrease sebum secretion and to stimulate hair growth.

### BACKGROUND OF THE INVENTION

Alopecia, or balding, is a common problem which medical science has yet to alleviate. While androgens are associated with balding, the physiological mechanism by which this hair loss occurs is not known. However, it is known that hair growth is altered in individuals afflicted with alopecia.

Hair does not grow continuously but undergoes cycles of activity involving periods of growth, rest, and shedding. The human scalp typically contains from 100,000 to 350,000 hair fibers or shafts, which undergo metamorphosis in three distinct stages:
(a) during the growth phase (anagen) the follicle (i.e. the hair root) penetrates deep into the dermis with the cells of the follicle dividing rapidly and differentiating in the process of synthesizing keratin, the predominant component of hair. In non-balding humans, this growth phase lasts from one to five years;
(b) the transitional phase (catagen) is marked by the cessation of mitosis and lasts from two to three weeks; and
(c) the resting phase (telogen) in which the hair is retained within the scalp for up to 12 weeks, until it is displaced by new follicular growth from the scalp below.

In humans, this growth cycle is not synchronized. An individual will have thousands of follicles in each of these three phases. However, most of the hair follicles will be in the anagen phase. In healthy young adults, the anagen to telogen ratio can be as high as 9 to 1. In Individuals with alopecia, this ratio is reduced to as low as 2:1.

Androgenetic alopecia arises from activation of an inherited sensitivity to circulating androgenic hormones. It is the most common type of alopecia. It affects both men (50%) and women (30%), primarily of Caucasian origin. Gradual changes in the width and length of the hair shaft are experienced over time and with increasing age, prematurely in some. Terminal hair is gradually converted to short, wispy, colorless vellus hair. As a consequence, men In their 20's and women In their 30's and 40's begin to notice their hair becoming finer and shorter. In males, most of the hair loss occurs at the crown of the head. Females experience a thinning over their entire scalp. As discussed above, the anagen to telogen ratio is reduced significantly, resulting in less hair growth.

Minoxidil, a potassium channel opener, promotes hair growth. Minoxidil is available commercially in the United States under the trademark, Rogaine^{®}. While the exact mechanism of action of minoxidil is unknown, its impact on the hair growth cycle is well documented. Minoxidil promotes the growth of the hair follicle and increase the period of time that the hair follicle is in the anagen phase (i.e., increases the anagen to telogen ratio).

While minoxidil promotes hair growth, the cosmetic efficacy of this growth can vary widely. For example, Roenigk reported the results of a clinical trial involving 83 males who used a topical solution of 3% minoxidil for a period of 19 months. Hair growth occurred in 55% of the subjects. However, only 20% of the subjects considered the growth to be cosmetically relevant. (Clin.Res., 33, No. 4, 914A, 1985). Tosti reported cosmetically acceptable re-growth In 18.1% of his subjects. (Dermatologica, 173, No. 3,136-138,1986). Thus, the need exists In the art for compounds having the ability produce higher rates of cosmetically acceptable hair growth in patients with alopecia.

DE 102 18 963 A1 discloses compounds of formula 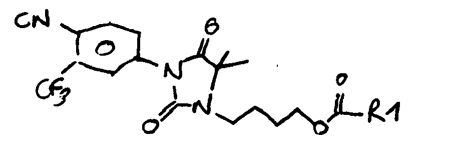 having anti-androgenic activity,

### SUMMARY OF THE INVENTION

In accordance with the present invention, a new class of benzonitriles has been discovered. These compounds, their salts, solvates, and prodrugs thereof, may be represented by Formula I below: 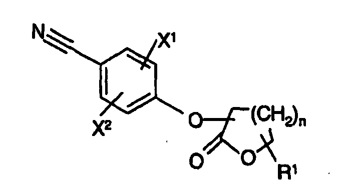 in which;
a) X¹ is chloro or trifluoromethyl and in located at the 2- or 6- position;
b) X² is absent, or is represented by halogen, cyano, C₁-C₆ alkoxy, haloalkoxy, or haloalkyl,
c) n is represented by an integer from 1 to 4,
d) R¹ is represented by a substituent selected from the group consisting of:
   i) hydrogen,
   ii) halogen,
   iii) cyano,
   iv) hydroxy,
   v) (C₁-C₁₂)alkyl, optionally substituted,
   vi) (C₂-C₁₂)alkenyl, optionally substituted,
   vii) (C₂-C₁₂)alkynyl, optionally substituted,
   viii) (C₃-C₁₀)cycloalkyl, optionally substituted,
   ix) (C₃-C₁₀) cycloalkyl(C₁-C₆)alkyl, in which the alkyl and cycloalkyl moieties may each be optionally substituted,
   x) (C₆-C₁₀)ary, optionally substituted,
   xi) (C₆-C₁₀)aryl (C₁-C₆)alkyl, in which the alkyl and aryl moieties may each be optionally substituted,
   xii) (CH₂)_{z}-SR³,
   xiii) (CH₂)_{z}-OR³,
   xiv) (CH₂)_{z}-NR³R⁴,
   xv) (CH₂)_{z}-COOR³,
   xvi) (CH₂)_{z}-CONR³R⁴,
   xvii) (CH₂)_{z}-NR⁴COR³, and
   xviii) (CH₂)_{z}OCOR³;
e) z is represented by an integer from 0 to 6,
f) R³ is represented by a substituent selected from the group consisting of hydrogen, (C₁-C₁₂)alkyl, (C₂-C₁₂)alkenyl, (C₂-C₁₂)alkynyl, optionally substituted (C₆-C₁₀)ary, and (C₆-C₁₀)aryl (C₁-C₆)alkyl, in which the alkyl and aryl moieties may each be optionally substituted and;
g) R⁴ is represented by a substituent selected from the group consisting of hydrogen, and (C₁-C₁₂)alkyl.

The compounds of Formula I are androgen receptor modulators. The compounds have affinity for the androgen receptor and will cause a biological effect by binding to the receptor. Typically, the compounds will act as antagonists. In selected embodiments they will act as partial agonists, full agonists, or tissue selective agonists. As androgen receptor modulators, the compounds can be used to treat, or alleviate, conditions associated with inappropriate activation of the androgen receptor. Examples of such conditions for antagonists include, but are not limited to, acne, excess sebum secretion, androgenic alopecia, hormone dependant cancers such as prostrate cancer, and hirsutism. Those compounds that are partial agonists, or full agonists, can be used to treat osteoporosis, hypogonadism, anemia, or to stimulate increases in muscle mass, especially in wasting diseases.

The invention is also directed to pharmaceutical compositions containing at least one of the compounds, in an amount effective to modulate activation of the androgen receptor. In a further embodiment, the invention is directed to an article of manufacture containing, at least one of the compounds packaged for retail distribution, in association with instructions advising the consumer on how to use the compound to alleviate a condition associated with inappropriate activation of the androgen receptor. An additional embodiment is directed to the use of a compound as a diagnostic agent to detect inappropriate activation of the androgen receptor.

In a further embodiment, the compounds are used topically to induce and/or stimulate hair growth and/or to slow down hair loss. The compounds may also be used topically in the treatment of excess sebum and/or of acne.

In a further embodiment the compounds can be used in livestock such as cattle, pigs, chickens, etc. The compounds will increase the growth rate, and enhance the lean meat to fat ratio in the animals, and improve feed efficiency.

### DETAILED DESCRIPTION OF THE INVENTION

The headings within this document are only being utilized expedite its review by the reader. They should not be construed as limiting the invention or claims in any manner.

### Definitions and Exemplification

As used throughout this application, including the claims, the following terms have the meanings defined below, unless specifically indicated otherwise. The plural and singular should be treated as interchangeable, other than the indication of number:
a. "halogen" refers to a chlorine, fluorine or bromine atom.
b. "C₁- C₆ alkyl" refers to a branched or straight chained alkyl group containing from 1 to 6 carbon atoms, such as methyl, ethyl, n-propyl, isopropyl, n-butyl, isobutyl, pentyl, etc.
c. "C₁- C₆ alkyl, optionally substituted" refers to a branched or straight chained alkyl group containing from 1 to 6 carbon atoms, such as methyl, ethyl, n-propyl, isopropyl, n-butyl, isobutyl, pentyl, etc. Such an alkyl group may be optionally substituted, in which up to 6 hydrogen atoms are replaced by a substituent selected from the group consisting of halogen, haloalkyl, hydroxy, thiol, cyano, and NR³R⁴ in which R³ and R⁴ are as defined above.
d. "C₁- C₁₂ alkyl, optionally substituted" refers to a branched or straight chained alkyl group containing from 1 to 12 carbon atoms, such as methyl, ethyl, n-propyl, isopropyl, n-butyl, isobutyl, hexyl, octyl, decyl, etc. Such an alkyl group may be optionally substituted, in which up to 8 hydrogen atoms are replaced by a substituent selected from the group consisting of halogen, haloalkyl, hydroxy, thiol, cyano, and NR³R⁴, in which R and R are as defined above.
e. "C₂-C₁₂ alkenyl optionally substituted" refers to a straight-chain or branched-chain hydrocarbon radical containing from 2 to 12 carbon atoms and 1, or more, carbon-carbon double bonds. Examples of alkenyl radicals include ethenyl, propenyl, 1,4-butadienyl, 1-hexenyl, 1,3-octadienyl and the like. Such an alkenyl group may be optionally substituted, in which up to 8 hydrogen atoms are replaced by a substituent selected from the group consisting of halogen, haloalkyl, hydroxy, thiol, cyano, and NR³R⁴, in which R³ and R⁴ are as defined above.
f. "C₂-C₁₂ aikynyi optionally substituted" refers to a straight-chain or branched-chain hydrocarbon radical containing from 2 to 12 carbon atoms and having 1, or more, carbon-carbon triple bonds. Examples of alkynyl radicals include ethynyl, propynyl, butynyl, octynyl, and the like. Such an alkynyl group may be optionally substituted, in which up to 8 hydrogen atoms are replaced by a substituent selected from the group consisting of halogen, hydroxy, haloalkyl, thiol, cyano, and -NR³R⁴, in which R³ and R⁴ are as defined above.
g. "haloalkyl" refers to a branched or straight chained alkyl group containing from 1 to 6 carbon atoms, in which at least one hydrogen atom is replaced with a halogen (i.e. C₁-C₆ haloalkyl). Examples of suitable haloalkyl's include chloromethyl, difluoromethyl, trifluoromethyl, 1-fluro-2-chloro-ethyl, 5-fluoro-hexyl, 3-difluro-isopropyl, 3-chloroisobutyl, etc.
h. "(C₁-C₂)alkyl substituted with one or more halogen atoms" refers to a straight chained alkyl group containing 1 or 2 carbon atoms, i.e., methyl or ethyl in which at least one hydrogen atom is replaced with a halogen (i.e. for example trifluromethyl, dichloromethyl, etc.).
i. "(C₁-C₂)alkoxy substituted with one or more halogen atoms" refers to a straight chained alkoxy group containing 1 or 2 carbon atoms, i.e., methoxy or ethoxy in which at least one hydrogen atom is replaced with a halogen (i.e. for example trifluoromethoxy, difluromethoxy, etc.)
j. "C₁-C₆ alkoxy" refers to a straight or branched chain alkoxy group containing from 1 to 6 carbon atoms, such as methoxy, ethoxy, n-propoxy, isopropoxy, n-butoxy, isobutoxy, pentoxy, etc.
k. "haloalkoxy" refers to a branched or straight chained alkoxy group containing from 1 to 6 carbon atoms, in which at least one hydrogen atom is replaced with a halogen (i.e. C₁-C₆ haloalkoxy). Examples of suitable haloalkoxy's include chloromethoxy, difluoromethoxy, trifluoromethoxy, 1-fluro-2-chloro-ethoxy, 5-fluoro-hexoxy, 3-difluroisopropoxy, 3-chloro-isobutoxy, etc.
I. "(C₆-C₁₀)aryl" optionally substituted means a cyclic, aromatic hydrocarbon containing from 6 to 10 carbon atoms. Examples of aryl groups include phenyl, naphthyl and biphenyl. Such an aryl moiety may be optionally substituted with up to 4 non-hydrogen substituents, each substituent is independently selected from the group consisting of halogen, cyano, hydroxy, (C₁-C₆)alkyl, (C₁-C₆)alkoxy, (C₁-C₂)alkyl substituted with one or more halogens, (C₁-C₂)alkoxy substituted with one or more halogens, SR⁵ and NR⁵R⁶. R⁵ and R⁶ are each independently represented by C₁-C₆ alkyl or hydrogen. These substituents may be the same or different and may be located at any position of the ring, that is chemically permissible.
m. "(C₃-C₁₀) cycloalkyl" optionally substituted refers to a saturated or partially saturated monocyclic, bicyclic or tricyclic alkyl radical wherein each cyclic moiety has 3 to 10 carbon atoms. Examples of cycloalkyl radicals include cyclopropyl, cyclobutyl, cyclopentyl, cyclohexyl, cyclooctyl, and the like. Such a cycloalkyl group may be optionally substituted, in which up to 4 hydrogen atoms are replaced by a substituent selected from the group consisting of halogen, cyano, hydroxy, (C₁-C₆)alkyl, (C₁-C₆)alkoxy, (C₁-C₂)alkyl substituted with one or more halogens, (C₁-C₂)alkoxy substituted with one or more halogens, SR⁵, and NR⁵R⁶, in which R⁵ and R⁶ are as defined above.
n. "androgen" refers to testosterone and its precursors and metabolites, and 5-alpha reduced androgens, including but not limited to dihydrotestosterone. Androgen refers to androgens from the testis, adrenal gland, and ovaries, as well as all forms of natural, synthetic and substituted or modified androgens.
o. "pharmaceutically acceptable" means suitable for use in mammals.
p. "salts" is intended to refer pharmaceutically acceptable salts and to salts suitable for use in industrial processes, such as the preparation of the compound.
q. "pharmaceutically acceptable salts" is intended to refer to either pharmaceutically acceptable acid addition salts" or "pharmaceutically acceptable basic addition salts" depending upon actual structure of the compound.
r. "pharmaceutically acceptable acid addition salts" is intended to apply to any non-toxic organic or inorganic acid addition salt of the base compounds represented by Formula I or any of its intermediates. Illustrative inorganic acids which form suitable salts include hydrochloric, hydrobromic, sulphuric, and phosphoric acid and acid metal salts such as sodium monohydrogen orthophosphate, and potassium hydrogen sulfate. Illustrative organic acids, which form suitable salts include the mono-, di-, and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxy-benzoic, phenylacetic, cinnamic, salicylic, 2-phenoxybenzoic, p-toluenesulfonic acid, and sulfonic acids such as methane sulfonic acid and 2-hydroxyethane sulfonic acid. Such salts can exist in either a hydrated or substantially anhydrous form. In general, the acid addition salts of these compounds are soluble in water and various hydrophilic organic solvents, and which in comparison to their free base forms, generally demonstrate higher melting points.
s. "pharmaceutically acceptable basic addition salts" is intended to apply to any non-toxic organic or inorganic basic addition salts of the compounds represented by Formula I, or any of its intermediates. Illustrative bases which form suitable salts include alkali metal or alkaline-earth metal hydroxides such as sodium, potassium, calcium, magnesium, or barium hydroxides; ammonia, and aliphatic, alicyclic, or aromatic organic amines such as methylamine, dimethylamine, trimethylamine, and picoline.
t. "prodrug" refers to compounds that are rapidly transformed in vivo to yield the parent compound of the above formulas, for example, by hydrolysis in blood. A thorough discussion is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are incorporated herein by reference.
u. "compound of Formula I", "compounds of the invention", and "compounds" are used interchangeably throughout the application and should be treated as synonoms.
v. "patient" refers to warm blooded animals such as, for example, guinea pigs, mice, rats, gerbils, cats, rabbits, dogs, monkeys, chimpanzees, stump tail macques, and humans.
w. "treat refers to the ability of the compounds to either relieve, alleviate, or slow the progression of the patient's disease (or condition) or any tissue damage associated with the disease.
x. "livestock" refers to animals suitable for human meat consumption. Examples include pigs, cattle, chickens, turkeys, rabbits, etc.
y. "isomer" means "stereoisomer" and "geometric isomer" as defined below.
z. "stereoisomer" means compounds that possess one or more chiral centers and each center may exist in the R or S configuration. Stereoisomers includes all diastereomeric, enantiomeric and epimeric forms as well as racemates and mixtures thereof.
aa. "geometric isomer" means compounds that may exist in cis, trans, anti, entgegen (E), and zusammen (Z) forms as well as mixtures thereof.

Certain of the compounds of the formula (I) may exist as geometric isomers. The compounds of the formula (I) may possess one or more asymmetric centers, thus existing as two, or more, stereoisomeric forms. The present invention includes all the individual stereoisomers and geometric isomers of the compounds of formula (I) and mixtures thereof.

In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention. The compounds may also exist in one or more crystalline states, i.e. polymorphs, or they may exist as amorphous solids. All such forms are encompassed by the claims.

All of the compounds of Formula I contain a phenyl ring. To further exemplify the invention, the numbering system for this ring and its substitution pattern is shown below:

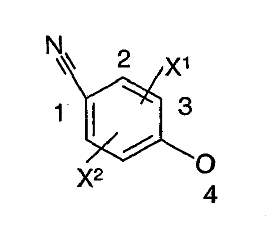

Position 1 of this phenyl ring is substituted with a cyano moiety as depicted above. Position 4 is substituted with an oxygen atom forming an ether moiety. The phenyl ring will be further substituted, as depicted by X¹, at position 2, 3, 5, or 6 with a halogen atom, a cyano group, a (C₁-C₆) alkoxy group, a haloalkoxy moiety or a haloalkyl moiety. Typically, it will be a halogen or haloalkyl moiety located at the 2- or 6-position. More typically, it will be trifluoromethyl located at the 2- or 6-position of the phenyl ring. Optionally, the phenyl ring may be substituted with a fourth (4^{th}) substituent, as indicated by X². X², if present, may be represented by a halogen atom, a cyano group, a (C₁-C₆) alkoxy group, a haloalkoxy moiety or a haloalkyl moiety.

The oxygen atom at the 4-position of the benzonitrile forms an ether linkage with a lactone as depicted blow:

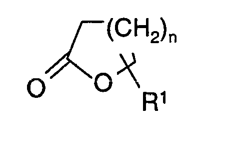

The number of carbon atoms in the lactone may vary, as indicated by n, which represents an integer from 1 to 4. Thus, the lactone may be a 5, 6, 7, or 8 membered ring. Examples of such lactones include dihydro-pyran-2-ones, tetrahydro-pyran-2-ones, dihydro-furan-2-ones, tetrahydro-furan-2-ones, etc.

The ether linkage may be bonded to any carbon atom of the lactone that is chemically permissible. For example, if the lactone is a furan-2-one, the ether may be attached to position 3, 4, or 5 of the lactone. Typically, the ether will be bonded to position 3 of the furan-2-one. If the lactone is a pyran-2-one, the ether may be bonded to position 3, 4, 5, or 6 of the lactone. Typically it will be bonded to the 3-position of the pyran-2-one.

The lactone may be optionally substituted with any of the substituents listed above for R¹. R¹ may represent up to 6 non-hydrogen substituents, if chemically permissible. These non-hydrogen substituents may be bonded to any carbon atom of the lactone, that is chemically permissible. A single carbon atom may be mono-substituted or di-substituted. If di-substituted, the relevant carbon atom may be substituted with the same, or differing substituents.

More specific embodiments of the invention include compounds of Formula I in which:
i) X¹ is chloro or trifluoromethyl and is located at the 2- or 6-position of the phenyl ring, X² is absent, n is 1 or 2, and R¹ is as defined above;
ii) X¹ is chloro or trifluoromethyl and is located at the 2- or 6-position of the phenyl ring, X² is absent, n is 1, and R¹ is as defined above;
iii) X¹ is chloro or trifluoromethyl and is located at the 2- or 6-position of the phenyl ring, X² is absent, n is 2, and R¹ is as defined above;
iv) X¹ is chloro or trifluoromethyl and is located at the 2- or 6-position of the phenyl ring, X² is absent, n is 1 or 2, and R¹ is selected from the group consisting of C₁-C₆ alkyl, C₁-C₆ alkoxy, halogen, haloalky, and haloalkoxy.
v) X¹ is trifluoromethyl and is located at the 2- or 6-position of the phenyl ring, X² is absent, n is 1, and R¹ is represented by a substituent selected from the group consisting of methyl, ethyl, trifluoromethyl, methoxy, and ethoxy.

More specific examples of compounds represented by Formula t include:
i) (±)-4-(4,4-dimethyl-2-oxo-tetrahydro-furan-3-yloxy)-2-trifluoromethyl-benzonitrile;
ii) (R)-(+)-4-(4,4-dimethyl-2-oxo-tetrahydro-furan-3-yloxy)-2-, trifluoromethyl-benzonitrile;
iii) (S)-(-)-4-(4,4-dimethyl-2-oxo-tetrahydro-furan-3-yloxy)-2-trifluoromethyl-benzonitrile;
iv) (±)-4-(2-oxo-tetrahydro-furan-3-yloxy)-2-trifluoromethyl-benzonitrile;
v) (+)-4-(2-oxo-tetrahydro-furan-3-yloxy)-2-trifluoromethyl-benzonitrile;
vi) (-)-4-(2-oxo-tetrahydro-furan-3-yloxy)-2-trifluoromethyl-benzonitrile;
vii) (±)-4-(5,5-dimethyl-2-oxo-tetrahydro-furan-3-yloxy)-2-trifluoromethyl-benzonitrile;
viii) (±)-4-(4-methoxy-2-oxo-tetrahydro-furan-3-yloxy)-2-trifluoromethyl-benzonitrile;
ix) (±)-4-(5-methoxy-4-trifluoromethyl-2-oxo-tetrahydro-furan-3-yloxy)-2-trifluoromethyl-benzonitrile;
x) (±)-4-(5-cyclohexyl-4-methyl-2-oxo-tetrahydro-furan-3-yloxy)-2-trifluoromethyl-benzonitrile
xi) (±)-4-(5-benzyl-4-fluoro-2-oxo-tetrahydro-furan-3-yloxy)-2-trifluoromethyl-benzonitrile;
xii) (±)-4-(5-cyano-4-methyl-2-oxo-tetrahydro-furan-3-yloxy)-2-trifluoromethyl-benzonitrile;
xiii) (±)-4-(4-methyl-2-oxo-tetrahydro-furan-3-yloxy)-2-trifluoromethyl-benzonitrile;
xiv) (±)-4-(4-fluoro-2-oxo-tetrahydro-furan-3-yloxy)-2-trifluoromethyl-benzonitrile
xv) (±)-4-(4-methoxy-2-oxo-tetrahydro-furan-3-yloxy)-2-trifluoromethyl-benzonitrile
xvi) (±)-4-(4-methoxy-2-oxo-tetrahydro-pyran-3-yloxy)-2-trifluoromethyl-benzonitrile
xvii) (±)-4-(2-oxo-tetrahydro-pyran-3-yloxy)-2-trifluoromethyl-benzonitrile;
xviii) (±)-4-(6-cyclopentyl-2-oxo-tetrahydro-pyran-3-yloxy)-2-trifluoromethyl-benzonitrile, and;
xix) (±)-4-(6-methyl-5-fluoro-4-methoxy-2-oxo-tetrahydro-pyran-3-yloxy)-2-trifluoromethyl-benzonitrile

### Synthesis

The compounds of Formula I can be prepared using methods known in the art for the preparation of ethers. The reader's attention is directed to European Patent Application Number 58932, published September 1, 1982 for a description of such reactions. Scheme I below provides an overview of one such technique:

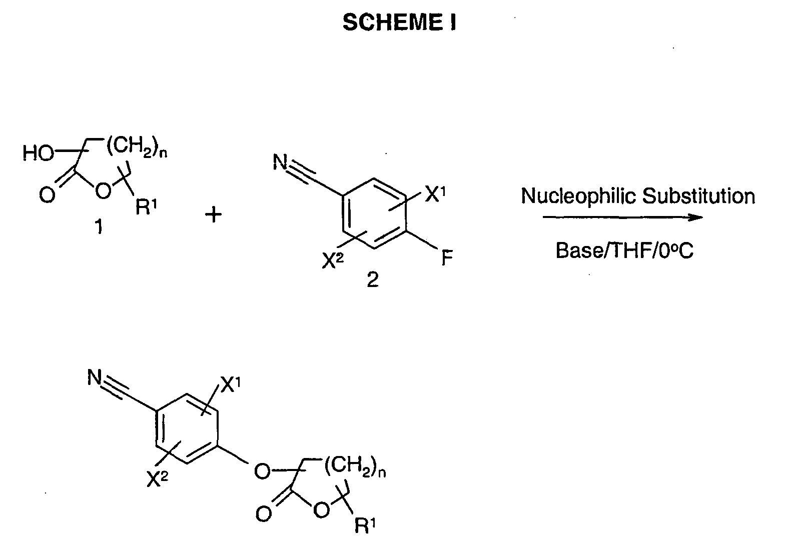

As depicted above, one of the starting materials is an alcohol as depicted by structure 1. R¹should be represented by the same substituent(s) as is desired in the final product. Likewise, n should be represented by the same integer as required in the final product. Such, lactones are known in the art. Many may be purchased from known commercial sources. Alternatively, they can be prepared as described in the literature.

The other starting material is a 4-fluoro-benzonitrile as depicted by structure 2. X¹ and X² should each be represented by the same substituent as desired in the final product. These benzonitriles are known in the art and may be synthesized as described by Japanese Patent Application Number 01097937.

The nucleophilic substitution depicted above may be carried out as is known in the art. The alcohol of structure 1 is contacted with a slight excess of a base, such as sodium hydride, potassium t-butoxide, etc., to produce an alkoxide ion. The reaction is carried out in an aprotic solvent, such as tetrahydrofuran, under an inert atmosphere (typically nitrogen) at a temperature of about 0°C. The alcohol is stirred with the base for a period of time ranging from 5 to 60 minutes.

One equivalent of the 4-fluoro-benzonitrile of structure 2 is then added to the reaction medium and the reactants are stirred for a sufficient period of time to allow the alkoxide ion to displace the fluorine from the benzonitrile. This typically takes from 30 minutes to 24 hours. The reaction is typically allowed to warm to room temperature.

The desired product of Formula I can be recovered by extraction, evaporation, or other techniques known in the art. It may then be optionally purified by chromatography, recrystallization, distillation, or other techniques known in the art.

As would be appreciated by those skilled in the art, some of the methods useful for the preparation of such compounds, as discussed above, may require protection of a particular functionality, e.g., to prevent interference by such functionality in reactions at other sites within the molecule or to preserve the integrity of such functionality. The need for, and type of, such protection is readily determined by one skilled in the art, and will vary depending on, for example, the nature of the functionality and the conditions of the selected preparation method. See, e.g., T.W. Greene, Protective Groups in Organic Synthesis, John Wiley & Sons, New York, 1991.

Some of the compounds of this invention are acidic and form salts with pharmaceutically acceptable cations. Some of the compounds of this invention are basic and form salts with pharmaceutically acceptable anions. All such salts are within the scope of this invention and they can be prepared by conventional methods such as combining the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate. The compounds are obtained in crystalline form according to procedures known in the art, such as by dissolution in an appropriate solvent(s) such as ethanol, hexanes or water/ethanol mixtures.

### Medical and Cosmetic Uses

The compounds of Formula I are androgen receptor modulators. They can be used to alleviate conditions associated with inappropriate activation of the androgen receptor. Compounds acting as androgen antagonists may be used to treat, or alleviate, hormone dependent cancers such as prostate carcinomas, benign hyperplasia of the prostate, acne, hirsutism, excess sebum, alopecia, hypertrichosis, precocious puberty, prostamegaly, virilization, and polycystic ovary syndrome. Compounds acting as partial agonists, or full agonists, may be used to treat, or alleviate, male hypogonadism, male sexual dysfunction (impotence, male dysspemtatogenic sterility), abnormal sex differentiation (male hermaphroditism), male delayed puberty, male infertility, aplastic anemia, hemolytic anemia, sickle cell anemia, idiopathic thrombocytopenic purpura, myelofibrosis, renal anemia, wasting diseases (post operative, malignant tumor, trauma, chronic renal disease, burn or AIDS induced), abatement of pain in terminal carcinoma of female genitalia, inoperable breast cancer, mastopathy, endometriosis, female sexual dysfunction, osteoporosis, wound healing and muscle tissue repair.

In order to exhibit the therapeutic properties described above, the compounds need to be administered in a quantity sufficient to modulate activation of the androgen receptor. This amount can vary depending upon the particular disease/condition being treated, the severity of the patient's disease/condition, the patient, the particular compound being administered, the route of administration, and the presence of other underlying disease states within the patient, etc. When administered systemically, the compounds typically exhibit their effect at a dosage range of from about 0.1 mg/kg/day to about 100 mg/kg/day for any of the diseases or conditions listed above. Repetitive daily administration may be desirable and will vary according to the conditions outlined above.

The compounds of the present invention may be administered by a variety of routes. They may be administered orally. The compounds may also be administered parenterally (i.e., subcutaneously, intravenously, intramuscularly, intraperitoneally, or intrathecally), rectally, or topically.

In a typical embodiment, the compounds are administered topically. Topical administration is especially appropriate for hirsutism, alopecia, acne and excess sebum. The dose will vary, but as a general guideline, the compound will be present in a dermatologically acceptable carrier in an amount of from about 0.01 to 50 w/w%, and more typically from about 0.1 to 10 w/w%. The dermatological preparation will be applied to the affected area from 1 to 4 times daily. "Dermatologically acceptable" refers to a carrier which may be applied to the skin or hair, and which will allow the drug to diffuse to the site of action. More specifically, it refers the site where inhibition of activation of an androgen receptor is desired.

In a further embodiment, the compounds are used topically to relieve alopecia, especially androgenic alopecia. Androgens have a profound effect on both hair growth and hair loss. In most body sites, such as the beard and pubic skin, androgens stimulate hair growth by prolonging the growth phase of the hair cycle (anagen) and increasing follicle size. Hair growth on the scalp does not require androgens but, paradoxically, androgens are necessary for balding on the scalp in genetically predisposed individuals (androgenic alopecia) where there is a progressive decline in the duration of anagen and in hair follicle size. Androgenic alopecia is also common in women where it usually presents as a diffuse hair loss rather than showing the patterning seen in men.

While the compounds will most typically be used to alleviate androgenic alopecia, the invention is not limited to this specific condition. The compounds may be used to alleviate any type of alopecia. Examples of non-androgenic alopecia include alopecia areata, alopecia due to radiotherapy or chemotherapy, scarring alopecia, stress related alopecia, etc. As used in this application, "alopecia" refers to partial or complete hair loss on the scalp.

Thus, the compounds can be applied topically to the scalp and hair to prevent, or alleviate balding. Further, the compound can be applied topically in order to induce or promote the growth of hair on the scalp.

In a further embodiment of the invention, a compound of Formula I is applied topically in order to prevent the growth of hair in areas where such hair growth is not desired. One such use will be to alleviate hirsutism. Hirsutism is excessive hair growth in areas that typically do not have hair (i.e. a female face). Such inappropriate hair growth occurs most commonly in women and is frequently seen at menopause. The topical administration of the compounds will alleviate this condition leading to a reduction, or elimination of this inappropriate, or undesired, hair growth.

The compounds may also be used topically to decrease sebum production. Sebum is composed of triglycerides, wax esters, fatty acids, sterol esters and squalene. Sebum is produced in the acinar cells of the sebaceous glands and accumulates as these cells age. At maturation, the acinar cells lyse, releasing sebum into the lumenal duct so that it may be deposited on the surface of the skin.

In some individuals, an excessive quantity of sebum is secreted onto the skin. This can have a number of adverse consequences. It can exacerbate acne, since sebum is the primary food source for *Propionbacterium acnes,* the causative agent of acne. It can cause the skin to have a greasy appearance, typically considered cosmetically unappealing.

Formation of sebum is regulated by growth factors and a variety of hormones including androgen. The cellular and molecular mechanism by which androgens exert their influence on the sebaceous gland has not been fully elucidated. However, clinical experience documents the impact androgens have on sebum production. Sebum production is significantly increased during puberty, when androgen levels are their highest. Anti-androgens, such as finasteride, have been shown to decrease androgen secretion. For additional information on sebum production and androgens role in skin metabolism, see Moshell et al, Progress in Dermatology, vol. 37, No. 4, Dec. 2003.

Thus, the compounds of formula I inhibit the secretion of sebum and thus reduce the amount of sebum on the surface of the skin. The compounds can be used to treat a variety of dermal diseases such as acne or seborrheic dermatitis.

In addition to treating diseases associated with excess sebum production, the compounds can also be used to achieve a cosmetic effect. Some consumers believe that they are afflicted with overactive sebaceous glands. They feel that their skin is oily and thus unattractive. These individuals can utilize the compounds of Formula I to decrease the amount of sebum on their skin. Decreasing the secretion of sebum will alleviate oily skin in individuals afflicted with such conditions.

In a further embodiment, those compounds acting as partial agonists, or full agonists, may be used to treat, or alleviate, osteoporosis. Osteoporosis is characterized by bone loss, resulting from an imbalance between bone resorption (destruction) and bone formation, which starts in the fourth decade and continues throughout life at the rate of about 1-4% per year (Eastell, Treatment of postmenopausal osteoporosis, New Eng. J. Med. 338: 736, 1998). In the United States, there are currently about 20 million people with detectable fractures of the vertebrae due to osteoporosis. In addition, there are about 250,000 hip fractures per year due to osteoporosis, associated with a 12%-20% mortality rate within the first two years, while 30% of patients require nursing home care after the fracture and many never become fully ambulatory again. In postmenopausal women, estrogen deficiency leads to increased bone resorption resulting in bone loss in the vertebrae of around 5% per year, immediately following menopause. Thus, first line treatment/prevention of this condition is inhibition of bone resorption by bisphosphonates, estrogens, selective estrogen receptor modulators (SERMs) and calcitonin. However, inhibitors of bone resorption are not sufficient to restore bone mass for patients who have already lost a significant amount of bone. The increase in spinal BMD attained by bisphosphonate treatment can reach 11 % after 7 years of treatment with alendronate. In addition, as the rate of bone turnover differs from site to site; higher in the trabecular bone of the vertebrae than in the cortex of the long bones, the bone resorption inhibitors are less effective in increasing hip BMD and preventing hip fracture. Therefore, osteoanabolic agents, which increase cortical/periosteal bone formation and bone mass of long bones, would address an unmet need in the treatment of osteoporosis especially for patients with high risk of hip fractures.

A number of studies demonstrate that androgens are osteoanabolic in women and men. Anabolic steroids, such as nandrolone decanoate or stanozolol, have been shown to increase bone mass in postmenopausal women. Beneficial effects of androgens on bone in post- menopausal osteoporosis are well documented in recent studies using combined testosterone and estrogen administration (Hofbauer, et al., Androgen effects on bone metabolism: recent progress and controversies, Eur. J. Endocrinol. 140, 271-286, 1999). Thus those compounds of Formula I exhibiting agonist or partial agonist activity may be used to treat, or alleviate, osteoporosis, including primary osteoporosis such as senile, postmenopausal and juvenile osteoporosis, as well as secondary osteoporosis, such as osteoporosis due to hyperthyroidism or Cushing syndrome (due to corticosteroid treatment), acromegaly, hypogonadism, dysosteogenesis and hypophosphatasemia. Other bone related indications amendable to treat from androgen agonists include osteoporotic fracture, childhood idiopathic bone loss, alveolar bone loss, mandibular bone loss, bone fracture, osteotomy, periodontitis, or prosthetic ingrowth.

Those compounds acting as agonists, or partial agonists, can also be used to stimulate muscle mass in patients afflicted with wasting diseases, such as AIDS, cancer cachexia, burns, renal disease, etc. Patients suffering from trauma, bedsores, age, etc. can also benefits from the anabolic effects of androgens.

### Co-Administration

In a further embodiment of the invention, the compounds of Formula I can be co-administered with other compounds to further enhance their activity, or to minimize potential side effects. For example, potassium channel openers, such as minoxidil, are known to stimulate hair growth and to induce anagen. Examples of other potassium channel openers include (3S,4R)-3,4-dihydro-4-(2,3-dihydro-2-methyl-3-oxopyridazin-6-yl)oxy-3-hydroxy-6-(3-hydroxyphenyl)sulphonyl-2,2,3-trimethyl-2H-benzo[b]pyran, diaxozide, and P1075 which is under development by Leo Pharmaceuticals. Such compounds can be co-administered with the compounds of Formula I to alleviate alopecia

Thyroid hormone is also known to stimulate hair growth. Synthetic thyroid hormone replacements (i.e., thyromimetics) have also been shown to stimulate hair growth. Such thyromimetics have been described in the literature previously. The reader's attention is directed to European Patent Application No. 1262177, the contents of which are hereby incorporated by reference, for a discussion of such compounds and their use to alleviate alopecia. One particular compound of interest is 2-{4-[3-(4-Fluoro-benzyl)-4-hydroxy-phenoxy]-3,5-dimethyl-phenyl}-2H-[1,2,4]triazine-3,5-dione. Such compounds can be co-administered with the compounds of Formula I to alleviate alopecia.

Anti-androgens can work by a number of different mechanisms. For example, some compounds block the conversion of testosterone to 5-α-dihydrotestosterone, which is responsible for the biological effect in many tissues. 5-Alpha-reductase inhibitors, such as finasteride, have been shown to stimulate hair growth and to decrease sebum production. Finasteride is commercially available from Merck under the trade name Propecia^{®}. Examples of other 5-α-reductase inhibitors include dutasteride (Glaxo Smithkline). Such compounds can be co-administered with the compounds of Formula I to alleviate alopecia and/or to decrease sebum production.

Protein kinase C inhibitors have also been shown to stimulate hair growth and induce anagen. Calphostin C, which is a selective inhibitor of protein kinase C, has been shown to induce anagen. Other selective protein kinase C inhibitors, such as hexadecylphosphocholine, palmitoyl-DL-carnitine chloride, and polymyxin B sulfate have also been shown to induce anagen. [Skin Pharmacol Appl Skin Physiol 2000 May-Aug;13(3-4):133-42]. Any such protein kinase C inhibitor can be co-administered with a compound of Formula I to alleviate alopecia.

lmmunophilins are a family of cytoplasmic proteins. Their ligands include cyclosporin, FK506, and rapamycin. They are derived from fungi and were developed primarily for their potent immunosuppressive properties. Cyclosporin binds to the proteins, cyclophilins, while FK506 and rapamycin bind to FK binding proteins (FKBPs). All of these compounds have been shown to stimulate hair growth and induce anagen. Any such immunophilin ligands can be co-administered with a compound of Formula I to alleviate alopecia.

Acyl CoA cholesterol acyl transferase (ACAT) inhibitors were initially evaluated for the treatment of elevated serum cholesterol. It was subsequently discovered that these compounds decrease sebum production (United States Patent No. 6,133,326). Any such ACAT inhibitor can be co-administered with a compound of formula l to decrease sebum production, alleviate oily skin, etc.

Antibiotics, such as tetracycline and clindamycin, have been used to alleviate acne. The antibiotic eradicates the microorganism, *Propionbacterium acnes,* leading to a reduction in the patient's acne. The compounds of Formula I can be co-administered with any antibiotic suitable for the treatment of acne.

Retinoids, such as isotretinoin, have been shown to decrease sebum production and are used to treat acne. These retinoids can be co-administered with a compound of Formula I in order to decrease sebum production and/or to treat acne.

Estrogen and progesterone have each been shown to decrease sebum production. These compounds, or any synthetic agonist of such compounds, may be co-administered with a compound of formula I in order to decrease sebum production.

As used in this application, co-administered refers to administering a compound of Formula I with a second medicinal, typically having a differing mechanism of action, using a dosing regimen that promotes the desired result. This can refer to simultaneous dosing, dosing at different times during a single day, or even dosing on different days. The compounds can be administered separately or can be combined into a single formulation. Techniques for preparing such formulations are described below.

### Formulations

If desired, the compounds can be administered directly without any carrier. However, to ease administration, they will typically be formulated into pharmaceutical carriers. Likewise, they will most typically be formulated into dermatological, or cosmetic carriers. In this application the terms "dermatological carrier" and "cosmetic" carrier are being used interchangeably. They refer to formulations designed for administration directly to the skin or hair.

Pharmaceutical and cosmetic compositions can be manufactured utilizing techniques known in the art. Typically an effective amount of the compound will be admixed with a pharmaceutically/cosmetically acceptable carrier.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations.

In another embodiment, the compounds of Formula I can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or non-aqueous pharmaceutically acceptable solvent, which may also contain suspending agents, sweetening agents, flavoring agents, and preservative agents as are known in the art.

For parenteral administration, the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art. When the compounds are being administered intrathecally, they may also be dissolved in cerebrospinal fluid as is known in the art.

The compounds of this invention will typically be administered topically. As used herein, topical refers to application of the compounds (and optional carrier) directly to the skin and/or hair. The topical composition according to the present invention can be in the form of solutions, lotions, salves, creams, ointments, liposomes, sprays, gels, foams, roller sticks, or any other formulation routinely used in dermatology.

Thus, a further embodiment relates to cosmetic or pharmaceutical compositions, in particular dermatological compositions, which comprise at least one of the compounds corresponding to Formula I above. Such dermatological compositions will contain from 0.001% to 10% w/w% of the compounds in admixture with a dermatologically acceptable carrier, and more typically, from 0.1 to 5 w/w% of the compounds. Such compositions will typically be applied from 1 to 4 times daily. The reader's attention is directed to Remington's Pharmaceutical Science, Edition 17, Mack Publishing Co., Easton, PA for a discussion of how to prepare such formulations.

The compositions according to the invention can also consist of solid preparations constituting cleansing soaps or bars. These compositions are prepared according to the usual methods.

The compounds can also be used for the hair in the form of aqueous, alcoholic or aqueous-alcoholic solutions, or in the form of creams, gels, emulsions or mousses, or alternatively in the form of aerosol compositions also comprising a propellant under pressure. The composition according to the invention can also be a hair care composition, and in particular a shampoo, a hair-setting lotion, a treating lotion, a styling cream or gel, a dye composition, a lotion or gel for preventing hair loss, etc. The amounts of the various constituents in the dermatological compositions according to the invention are those conventionally used in the fields considered.

The medicinal and cosmetics containing the compounds of the invention will typically be packaged for retail distribution (i.e. an article of manufacture). Such articles will be labeled and packaged in a manner to instruct the patient how to use the product. Such instructions will include the condition to be treated, duration of treatment, dosing schedule, etc.

The compounds of Formula I may also be admixed with any inert carrier and utilized in laboratory assays in order to determine the concentration of the compounds within the serum, urine, etc., of the patient as is known in the art. The compounds may also be used as a research tool.

### Use in Livestock

In addition to the therapeutic and cosmetic uses described above, the compounds may also be used to promote the growth of animals, especially livestock. The compounds will increase the rate at which the animals gain weight, increase the leanness of the resulting meat and improve the efficiency of feed utilization. This may be accomplished by administering an effective amount of a compound of Formula I to an animal receiving adequate nutrition to support growth (i.e. sufficient calories, amino acids, vitamins, minerals, essential fats, etc).

To simplify administration, the compound is typically mixed with animal feeds or prepared in the form of an animal-feed premix, concentrate, or supplement which can be blended with animal feeds. Regardless of the procedure selected, the compound will typically be present at levels of from about 0.05 to 500 ppm in the feed.

Animal-feed premixes, supplements or concentrates can be prepared by mixing on a weight basis about 0.5 to 50% of a compound with about 50 to 99.5% of an edible diluent. Diluents suitable for use in the manufacture of animal-feed supplements, concentrates, and premixes include the following: corn meal, soybean meal, bone meal, alfalfa meal, cottonseed oil meal, urea, molasses, and other similar materials. Use of the diluents in feed supplements, concentrates, and premixes improves uniformity of distribution of the active ingredient in the finished feed.

Feeds for swine, cattle, sheep, and goats typically contains about 0.05 to 400 grams of active ingredient per ton of feed. Poultry and domestic-pet feeds range from about 0.05 to 400 grams per ton of feed.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention. The following examples and biological data is being presented in order to further illustrate the invention. This disclosure should not be construed as limiting the invention in any manner.

### EXAMPLES

### EXAMPLE 1

### (±)-4-(4,4-Dimethyl-2-oxo-tetrahydro-furan-3-yloxy)-2-trifluoromethyl-benzonitrile)

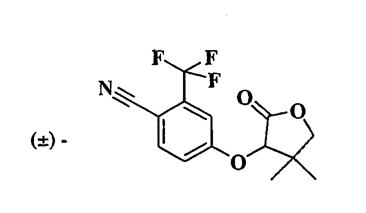

Sodium hydride/mineral oil (60% dispersion, 11g, 270 mmol) in dried tetrahydrofuran (50 mL)_was added to a cold (-15 °C) stirring solution consisting of 3-hydroxy-4,4-dimethyl-dihydro-furan-2-one(37.1 g, 281 mmol) in anhydrous tetrahydrofuran (90 mL). The reaction mixture was diluted with the addition of more anhydrous tetrahydrofuran (100 mL). To this stirring mixture after cessation of hydrogen gas liberation was added via canula a solution consisting of 4-fluoro-2-(trifluoromethyl) benzonitrile (55.4 g, 289 mmol) in anhydrous tetrahydrofuran (50 mL). The reaction mixture was allowed to stir overnight, whilst gradually warming to room temperature. Ethyl acetate (100 ml) was added and the solution was washed with saturated aqueous ammonium chloride, water, and twice with saturated aqueous sodium chloride. The organic layer was dried over anhydrous magnesium sulfate, filtered and concentrated. The crude solid (86.72g) was recrystallised from ethanol to afford 65.47 g (79.45 % yield) of a white crystalline solid;¹H NMR (400MHz; CDCl3) δ 7.76 (d, 1H, J=8.5 Hz), 7.42 (d, 1H, J=2.7 Hz), 7.30 (dd, 1H, J=8.5, 2.4 Hz), 4.69 (s, 1H), 4.15 (d, 1H, J=9.0 Hz), 4.09 (d, 1H, J=9.0 Hz), 1.27 (s, 3H), 1.27 (s, 3H); ¹⁹F NMR (376MHz; CDCl3) δ-62.70 (s, 3F); MS (APCI+) 341.1 (M+1+acetonitrile).

### EXAMPLE 2

### (R)-(+)-4-(4,4-Dimethyl-2-oxo-tetrahydro-furan-3-yloxy)-2-trifluoromethyl-benzonitrile

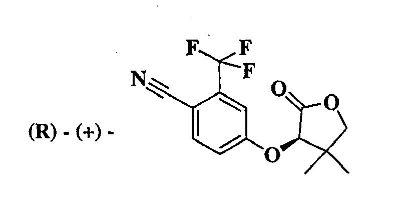

The enantiomers of (±)- 4-(4,4-dimethyl-2-oxo-tetrahydro-furan-3-yloxy)-2-trifluoromethyl-benzonitrile (Example 1) were separated by chiral HPLC (Chiralcel AD, 250x4.6 mm; mobile phase: 1:1 hexanes-isopropanol; flow rate: 0.7 mL/min.)
12.57 g (21 % column recovery); melting point 89.9-90.7 °C; [α]₅₈₉²⁵: +191.5 °; ¹⁹F NMR (376MHz; CDCl3) δ -62.70 (s, 3F); MS (APCI+) 341.1 (M+1+acetonitrile); microanalysis for C₁₄H₁₂F₃NO₃ (Theoretical/Found): C 56.19/56.25; H 4.04/3.86; N 4.68/4.67; F 19.05/18.64.

### EXAMPLE 3

### (S)-(-)-4-(4,4-Dimethyl-2-oxo-tetrahydro-furan-3-yloxy)-2-trifluoromethyl-benzonitrile

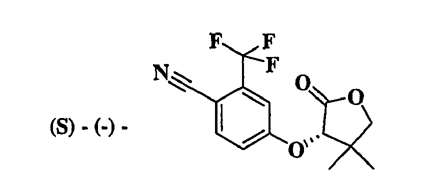

The enantiomers of (±)- 4-(4,4-dimethyl-2-oxo-tetrahydro-furan-3-yloxy)-2-trifluoromethyl-benzonitrile (Example 1) were separated by chiral HPLC (Chiralcel AD, 250x4.6 mm; mobile phase: 1:1 hexanes-isopropanol; flow rate: 0.7 mL/min.)
325 mg (29.8 % column recovery); melting point 83.1-84.4 °C;
[α]₅₈₉²⁵: -184.2 °; ¹⁹F NMR (376MHz; CDCl3) δ -62.70 (s, 3F); MS (APCI-) 298.0 (M-1); microanalysis for C₁₄H₁₂F₃NO₃ (Theoretical/Found): C 56.19/56.23; H 4.04/3.81; N 4.68/4.65; F 19.05/19.37.

### EXAMPLE 4

### (±)-4-(2-Oxo-tetrahydro-furan-3-yloxy)-2-trifluoromethyl-benzonitrile

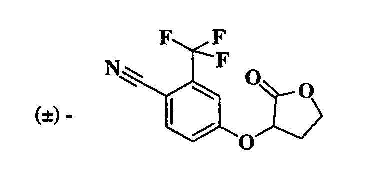

Sodium hydride (60% dispersion in mineral oil, 0.44 g, 18.34 mmol) was added in portions to a cold (-10 °C) stirring solution consisting of 2-hydroxy-γ-butyrolactone (1.29 g, 12.6 mmol) in tetrahydrofuran (25 mL). The reaction mixture was stirred for about 40 minutes before direct addition of the solid 4-fluoro-(2-trifluoromethyl)-benzonitrile (2.0 g, 11.0 mmol). The reaction mixture was allowed to gradually warm to room temperature overnight. Water was added and the product was extracted into ethyl acetate. The organic layer was washed with saturated aqueous sodium chloride, was dried over anhydrous magnesium sulfate, and was filtered and concentrated. The crude product was purified by flash chromatography. Elution with a gradient of 25-50% ethyl acetate in hexanes afforded a white solid. The product was recrystallized from ethyl acetate-hexanes to obtain 0.45 g (16 % yield) of a white crystalline solid; melting point 120 °C; ¹H-NMR (400 MHz; CDCl₃) δ 7.79 (d, 1H, J=8.5 Hz), 7.43 (d, 1H, J=2.4 Hz), 7.33 (dd, 1H, J=8.5,2.4 Hz), 5.08 (t, 1H, J=7.8 Hz), 4.57 (m, 1H), 4.43 (m, 1H), 2.78 (m, 1H), 2.56 (m, 1H); ¹⁹F-NMR (376 MHz; CDCl₃) δ -62.72 (s, 3F); MS (APCI) 270.0 (M-1); microanalysis for C₁₂H₈F₃O₃N (Theoretical/Found): C, 53.15/53.01; H, 2.97/2.81; N, 5.16/4.97; F, 21.02/21.24.

### EXAMPLE 5

### (+)-4-(2-Oxo-tetrahydro-furan-3-yloxy)-2-trinuoromethyl-benzonitrile

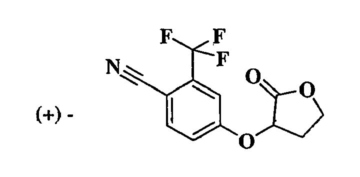

The enantiomers of (±)-4-(2-oxo-tetrahydro-furan-3-yloxy)-2-trifluoromethyl-benzonitrile (Example 4) were separated by chiral HPLC (Chiralcel AD, 250x4.6 mm; mobile phase: 20:80 ethanol-hexanes; flow rate: 0.8 mL/min.)
[α]₅₈₉²⁵ (CH₂Cl₂): -164 °; melting point 107-108 °C; MS (APCI-) 270.0 (M-1); microanalysis for C₁₂H₈F₃O₃N(Theoretical/Found): C, 53.15/52.98; H, 2.97/3.01; N, 5.16/4.97; F, 21.02/21.51.

### EXAMPLE 6

### (-)-4-(2-Oxo-tetrahydro-furan-3-yloxy)-2-trifluoromethyl-benzonitrile

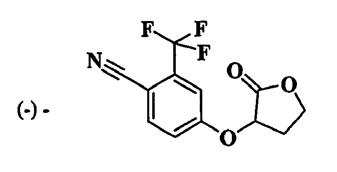

The enantiomers of (±)-4-(2-oxo-tetrahydro-furan-3-yloxy)-2-trifluoromethyl-benzonitrile (Example 4) were separated by chiral HPLC (Chiralcel AD, 250x4.6 mm; mobile phase: 20:80 ethanol-hexanes; flow rate: 0.8 mL/min.)
[α]₅₈₉²⁵ (CH₂Cl₂): +170 °; melting point 107-108 °C; MS (APCI-) 270.0 (M-1); microanalysis for C₁₂H₈F₃O₃N(Theoretical/Found): C, 53.15/52.77; H, 2.97/2.88; N, 5.16/4.97; F, 21.02/20.46.

### EXAMPLE 7

The compounds of Formula I have affinity for the androgen receptor. This affinity has been demonstrated for selected compounds using the human receptor. The description below describes how the assay was carried out.

Competitive binding analysis was performed on baculovirus/Sf9 generated hAR extracts in the presence or absence of different concentrations of test agent and a fixed concentration of ³H-dihydrotestosterone (³H-DHT) as tracer. This binding assay method is a modification of a protocol previously described (Liao S. , et. al. J. Steroid Biochem. 20:11-17 1984). Briefly, progressively decreasing concentrations of compounds are incubated in the presence of hAR extract (Chang et al. P.N.A.S. Vol. 89, pp. 5546-5950, 1992), hydroxylapatite, and 1 nM ³ H-DHT for one hour at 4°C. Subsequently, the binding reactions are washed three times to completely remove excess unbound ³H-DHT. hAR bound ³H-DHT levels are determined in the presence of compounds (i.e. competitive binding) and compared to levels bound when no competitor is present (i.e. maximum binding). Compound binding affinity to the hAR is expressed as the concentration of compound at which one half of the maximum binding is inhibited. Table I below provides the results that were obtained for selected compounds (reported data is the mean of multiple tests as shown below)

**Table I**

| Example | ARB (IC₅₀) nM |
|---|---|
| 1 | 122 (c) |
| 2 | 71 (a) |
| 3 | >10000 (a) |
| 4 | 5849 (a) |
| 5 | 9804 (a) |
| 6 | 1666 (a) |

| | |
|---|---|
| a - mean of 2 tests b - mean of 3 tests c - mean of 4 tests ND - not determined | |

### Example 8

The compounds ability to antagonize the effects of androgen on the androgen receptor were determined in a whole cell assay as described immediately below.

### Experimental procedure for AR antagonist cell assay

**Cell line:** MDA-MB453-MMTV clone 54-19. This cell line is a stable transfected cell line with MDA-MB453 cell background (a human breast tumor cell line expressing androgen receptor). A MMTV minimal promoter containing ARE was first cloned in front of a firefly luciferase reporter gene. Then the cascade was cloned into transfection vector pUV120puro. Electroporation method was used for transfecting MDA-MB-453 cell. Puromycin resistant stable cell line was selected.

### Cell culture media and reagents:

**Culture medium:** DMEM (high glucose, Gibco cat #: 11960-044), 10%FBS, and 1% L-glutamine
**Plating medium:** DMEM (phenol red free), 10% charcoal treated HyClone serum, 1% L-glutamine
**Assay medium:** DMEM (phenol red free), 1% charcoal treated HyClone serum, 1% L-glutamine, and 1% penicillin/streptomycin
**3X luciferase buffer:** 2% beta-mercaptoethanol, 0.6% ATP, 0.0135% luciferine in cell lysis buffer

### Assay procedure:

1. Cells are maintained in culture medium, splitting cells when they reach 80-90% confluence
2. To test compounds; 10,000 cells/well are plated to opaque 96 cell culture plate in 100 ul/well plating medium, culture for overnight at 37°C in cell culture incubator
3. Carefully remove plating medium, then add 80 ul/well of pre-warmed assay medium, add 10 ul/well testing compound (final concentration at) 1000 nM, 200 nM, 40 nM, 8 nM, 1.6 nM, and 0.32 nM), incubate at 37°C for 30 minutes
4. Add 10 ul/well freshly prepared DHT (final concentration at 100 pM) to each well, incubate at 37°C for 17 hr (overnight)
5. Add 50 ul/well 3X luciferase buffer, incubate at room temperature for 5 minutes, then count on Luminometer
The fold induction over background by 100 pM DHT in the absence of testing compounds is standardized as 100% and experimental result is expressed as percentage of inhibition by testing compounds.

The results are described below in Table II. The results are reported as the mean of multiple tests as described below (the numbers of tests are indicated in the footnote). N.D. denotes that the compound was not tested.

**Table II**

| Example | ARCell (IC₅₀) nM |
|---|---|
| 1 | 60 (a) |
| 2 | 47 (a) |
| 3 | >1000 (a) |
| 4 | ND |
| 5 | ND |
| 6 | ND |

| | |
|---|---|
| a - mean of 2 tests b - mean of 3 tests c - mean of 4 tests ND - not determined | |

### Example 9

### Animal Model for Inhibition of Sebum Production

Luderschmidt et al describes an animal model for testing whether compounds are capable of modulating sebum secretion. Arch. Derm. Res. 258, 185-191 (1977). This model uses male Syrian hamsters, whose ears contain sebaceous glands. The products of Examples 1 and 2 were screened in this model.

Testing for sebum inhibition was carried out in the following manner. Male Syrian hamsters aged 9 to 10 weeks were introduced into the laboratory environment and acclimated for 2 weeks prior to use in the study. Each group consisted of 5 animals and run in parallel with vehicle and positive controls. Prior to administration, a sufficient quantity each compound was dissolved in 1 mL of a solvent consisting of transcutol, propylene glycol, and ethanol (2/2/6 v/v/v) to achieve the final concentration described in Table III.

Animals were dosed topically twice daily, five days a week, for 4 weeks. Each dose consisted of 25 micro liters of vehicle control or drug. The dose was applied to the ventral surfaces of both the right and left ears. All animals were sacrificed approximately 18-24 hours after the final dose. The right ears were collected from each animal and used for sebum analysis.

The ears were prepped for HPLC analysis in the following manner. One 8mm distal biopsy punch was taken, just above the anatomical "V" mark in the ear to normalize the sample area. The punch was pulled apart. The ventral biopsy surface (the area where the topical dose was directly applied to the sebaceous glands) was retained for testing and the dorsal surface of the biopsy punch was discarded.

Tissue samples were blown with N₂ gas and stored at -80°C under nitrogen until HPLC analysis. In addition to ear samples, an aliquot of each drug and vehicle (at least 250ul) was also stored at -80°C for inclusion in the HPLC analysis.

HPLC analysis was carried out on an extract of the tissue sample. Tissue samples were contacted with 3ml of solvent (a 4:1 admixture of 2,2,4-trimethylpentane and isopropyl alcohol). The mixture was shaken for 15 minutes and stored overnight at room temperature, protected from light. The next morning 1 milliliter of water was added to the sample and shaken for 15 minutes. The sample was then centrifuged at approximately 1500rpm for 15 minutes. Two ml of the organic phase (top layer) was transferred to a glass vial, dried at 37°C, under nitrogen, for approximately 1 hour, and then lyophilized for approximately 48 hours. The samples were then removed from the lyophilizer and each vial was reconstituted with 600µl of solvent A (trimethylpentane/tetrahydrofuran (99:1). The samples were then recapped and vortexed for 5 minutes.

200µl of each sample was then transferred to a pre-labeled 200µl HPLC vial with 200 µL glass inserts. The HPLC vials were placed in the autosampler tray for the Agilent 1100 series HPLC unit. The Agilent 1100 HPLC system consisted of a thermostated autosampler, a quarternary pump, a column heater, and an A/D interface module. All components were controlled by Agilent ChemStation software. A Waters Spherisorb S3W 4.6x100 mm analytical column was maintained at 30°C by the Agilent column heater unit. The HPLC autosampler was programmed to maintain the sample temperature at 20C throughout the run.

10uL of each sample was injected in triplicate into the column. Two solvents were used for the solvent gradient. Solvent A was an admixture of trimethylpentane and tetrahydrofuran (99:1). Solvent B was ethylacetate. The gradient utilized is described in the table below:

| Time (min) | Solv A (%) | Solv B (%) | Flow (mL/min) |
|---|---|---|---|
| 0 | 99 | 1 | 2 |
| 2 | 96 | 4 | 2 |
| 6 | 60 | 40 | 2 |
| 7 | 5 | 95 | 2 |
| 10 | 5 | 95 | 2 |
| 10.1 | 99 | 1 | 2 |

The Sedex 75 Evaporative Light Scattering Detector (ELSD) was operated at 45°C with a gain of 5, and N₂ pressure maintained at 3.1 bar. Analog signal obtained by the instrument was sent to the Agilent A/D interface module where it was converted to a digital output. The conversion was based on a 10000 mAU/volt set point and the data rate was set at 10Hz (0.03 min). The resulting digital output was then feed into the Agilent ChemStation software for integration of the peak area.

The results of the HPLC analysis are reported below in Table III. The results are reported as the reduction in cholesterol ester (CE) and wax ester (WE) production, when compared to the vehicle control. A negative value reflects an increase in sebum, whereas a positive reflects a decrease.

**Table III**

| Example # | Conc. of Compound (% w/v) | % CE reduction | % WE reduction | Sum of WE & WE |
|---|---|---|---|---|
| 1 | 1% | 55 | 73 | 128 |
| 2 | 3% | 79 | 92 | 171 |

### EXAMPLE 10

### Animal Model for Androgenetic Alopeica

As described above, alopecia is a problem that medical science has devoted considerable resources to. As with any disease process, animal models have been developed to allow scientists to screen compounds for their potential relative efficacy. Those compounds showing the greatest efficacy in these animal models are considered for further study in humans. Two different animal models have been developed to date for alopecia. The first is the telogen conversion assay, which uses female C3H/HeN mice. The second model uses stump-tailed macaques, which are monkeys that suffer from androgenetic alopecia.

The telogen conversion assay measures the potential of a compound to convert the resting stage of the hair growth cycle ("telogen") to the active stage of the hair growth cycle ("anagen") in mice. This assay takes advantage of the fact that the fur (i.e. hair) of 7-week-old C3H/HeN mice is in the telogen phase. This phase continues until about 75 days of age. In this assay, selected areas of the mice are shaved, contacted with a test agent, or a control, and the difference in the rate of hair growth is measured (i.e. induction of the anagen phase). The first sign of anagen is the darkening of skin color as melanocytes in the follicles start to synthesize melanin, in preparation for the production of pigmented hairs. This model has a number of advantages. This includes the ready availability of female CH3HeN mice, the ability to screen large numbers of compounds quickly, and the ease of housing and handling such animals.

The primary disadvantage of this model is its lack of androgenetic dependency. While the exact cause of human baldness is not known, it is well documented that androgens induce a regression of hair follicles in the scalp. This post adolescent regressive change is a fundamental cause of male pattern baldness, (i.e. "androgenetic alopecia). This phenomenon occurs in both men and women who have inherited the genetic trait for alopecia, as mentioned previously. For a more detail discussion of the effects of androgens on human scalps, the readers attention is directed to Trueb, RM, Molecular Mechanisms of Androgenic Alopecia, Exp. Gerontology, 2002, 27:981-990.

Researchers looked for other animals whose hair growth was similar to that of humans. These lead researchers to stump-tailed macaques. These primates also suffer from androgenetic alopecia. Essentially all post adolescent macaques, in both sexes, exhibit the development of baldness. Like the development of male pattern baldness in humans, androgens are an indispensable triggering factor in macaque baldness. Thinning of the frontal scalp hairs begins to appear around the same age (4 years) when serum levels of testosterone become drastically elevated in male animals. Although the elevation of testosterone in females is approximately one tenth that of the male level, there is no difference in the incidence and the age of onset of baldness between male and female stump-tailed macaques. Topical application of anti-androgens have reversed this baldness in animals of both sexes (Pan, H J et al, Evaluation of RU58841 as an anti-androgen in prostate PC3 cells and a topical anti-alopecia agent in the bald scalp of stump tailed macaques. Endocrine 1998; 9:39-43).

While this model is a significant improvement over the telogen conversion assay as a model for human baldness, it suffers from a number of practical disadvantages. The macaques are expensive, relatively rare, labor intensive to maintain, and require long wash out periods between testing. Thus, the macaque is not a practical model for screening large numbers of compounds

It has been discovered that male C3H/HeN mice may be used in the telogen conversion assay, when evaluating anti-androgen test compounds. Thus, the model relates to a modification of the existing telogen conversion assay. Male C3H/HeN mice approximately 7 weeks old are utilized. These animals are also uniformly in telogen, like their female counterparts. However, once shaven, the androgens inherently present in these male mice inhibit the conversion of the hair follicles to the anagen phase. An anti-androgen will block this androgenic effect and the follicles will convert to anagen, like their female counterparts.

### EXAMPLE 10A

The compound described in Example 1 was submitted for further testing utilizing the modified telogen conversion assay, described above. The testing was carried out in the following manner.

Male C3H/HeN mice, 6 to 7 weeks old (Charles River Laboratories, Raleigh, NC) were used for the study. Fur was clipped from the dorsal region of the mice prior to initiation of the study. Only mice with pink skin, a visual indication of the telogen phase, were selected for inclusion in the study.

The test compound was dissolved in a vehicle consisting of transcutol, propylene glycol and ethanol (2/2/6 v/v/v) to achieve a concentration of 1 % w/v . The relevant dose was applied topically to the clipped dorsal region of the mice in one test group (7-10 mice) in a volume of 20 µl/cm². A second group of animals received only the vehicle to serve as a control. Treatments were applied twice daily for 4 weeks.

The treatment area was observed and graded every other day for signs of hair growth. The hair growth response was quantified by recording, for each animal, the day on which signs of hair growth first appeared over the treated area. The first sign of anagen was the darkening of skin color as melanocytes in the follicles started to synthesize melanin in preparation for the production of pigmented hairs. The mice were observed for 35 days or longer.

Anagen was initiated in the test group prior to its occurrence in the vehicle control group, as shown below in Figure 1.

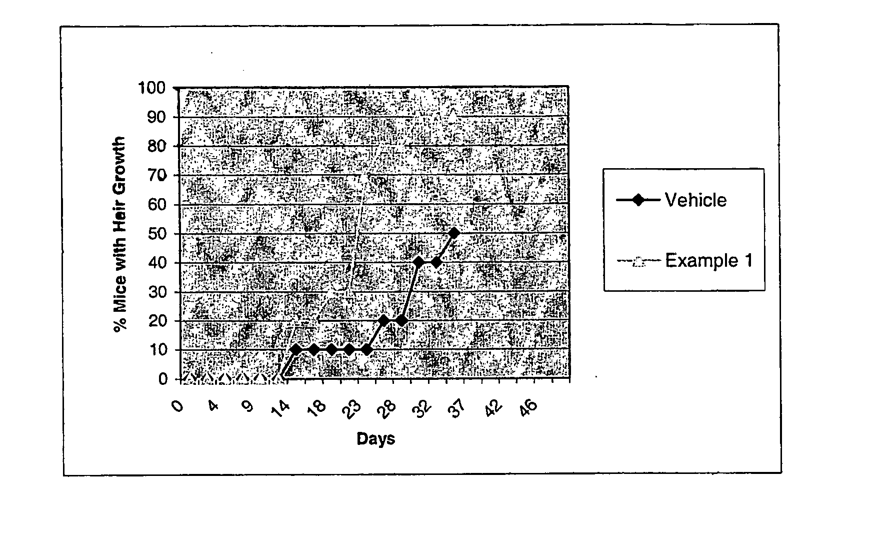

## Claims

1. A compound of the formula: 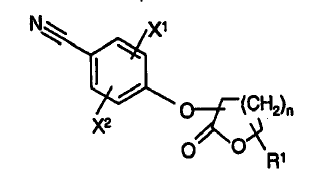 or a salt, thereof, in which;
a) X¹ is chloro or trifluoromethyl and is located at the 2- or 6-position;
b) X² is absent, or is represented by halogen, cyano, C₁-C₈ alkoxy, haloalkoxy, or haloalkyl;
c) n is represented by an integer from 1 to 4;
d) R¹ is represented by a substituent selected from the group consisting of:
i. hydrogen
ii. halogen,
iii. cyano,
iv. hydroxy,
v. (C₁-C₁₂)alkyl, optionally substituted,
vi. (C₂-C₁₂)alkenyl, optionally substituted,
vii. (C₂-C₁₂)alkynyl, optionally substituted,
viii. (C₃-C₁₀)cycloalkyl, optionally substituted,
ix. (C₃-C₁₀) cycloalkyl(C₁-C₆)alkyl, in which the alkyl and cycloalkyl moieties may each be optionally substituted,
x. (C₆-C₁₀)aryl, optionally substituted,
xi. (C₆-C₁₀)aryl (C₁-C₆)alkyl, in which the alkyl and aryl moieties may each be optionally substituted
xii. (CH₂)_{z}-SR¹,
xiii. (CH₂)_{z}-O-R¹,
xiv. (CH₂)_{z}-NR¹R²,
xv. (CH₂)_{z}-COOR³
xvi. (CH₂)_{z}-CONR³R⁴,
xvii. CH₂)_{z}-NR⁴COR³, and
xviii. (CH₂)_{z}OCOR³,
d) z is represented by an integer from 0 to 6,
e) R³ is represented by a substituent selected from the group consisting of hydrogen, (C₁-C₁₂)alkyl, (C₂-C₁₂)alkenyl, (C₂-C₁₂)alkynyl, optionally substituted (C₆-C₁₀)ary, and (C₆-C₁₀)aryl (C₁-C₆)alkyl, in which the alkyl and aryl moieties may each be optionally substituted and;
f) R⁴ is represented by a substituent selected from the group consisting of hydrogen or (C₁-C₁₂)alkyl.

2. A compound according to claim 1 in which n is 1.

3. A compound according to claim 1 or 2 which R¹ is selected from the group consisting of C₁-C₆ alkyl, C₁-C₆ alkoxy, halogen, haloalky, and haloalkoxy.

4. A compound according to claim 1 selected from the group consisting of
i) (±)-4-(4,4-dimethyl-2-oxo-tetrahydro-furan-3-yloxy)-2-triftuoromethyl-benzonitrile;
ii) (R)-(+)-4-(4,4-dimethyl-2-oxo-tetrahydro-furan-3-yloxy)-2-trifluoromethyl-benzonltrile;
iii) (S)-(-)-4-(4,4-dimethyl-2-oxo-tetrahydro-furan-3-yloxy)-2-trifluoromethyl-benzonitrile;
iv) (±)-4-(2-oxo-tetrahydro-furan-3-yloxy)-2-trifluoromethyl-benzonitrile;
v) (+)-4-(2-oxo-tetrahydro-furan-3-yloxy)-2-trifluoromethyl-benzonitrile, and;
vi) (-)-4-(2-oxo-tetrahydro-furan-3-yloxy)-2-trifluoromethyl-benzonitrile;

5. (R)-(+)-4-(4,4-dimethy)-2-oxo-tetrahydro-furan-3-yloxy)-2-trifluoromethyl-benzonitrile, or a pharmaceutically acceptable salt thereof.

6. A compound according to any one of claims 1 to 5 as a medicine.

7. Use of a compound according to any one of claims 1 to 5 in the manufacture of a medicament for alleviating a condition selected from the group consisting of hormone dependent cancers, benign hyperplasia of the prostate, acne, hirsutism, excess sebum, alopecia, premenstrual syndrome, lung cancer, precocious puberty, osteoporosis, hypogonadism, age-related decrease In muscle mass, and anemia.

8. A pharmaceutical composition comprising a compound according to any one of claims 1 to 5 in admixture with one or more pharmaceutically acceptable excipients.

9. A topical pharmaceutical formulation comprising a compound according to any one of claims 1 to 5 in admixture with or more pharmaceutically acceptable excipients suitable for dermal application.

10. An article of manufacture comprising a compound according to any one of claims 1 to 5, packaged for retail distribution, which advises a consumer how to utilize the compound to alleviate a condition selected from the group consisting of acne, alopecia, and oily skin.

11. Use of a compound according to any one of claims 1 to 5 in the manufacture of a medicament for alleviating a condition selected from the group consisting of alopecia, excess sebum and hirsutism.

12. (±)-4-(2-oxo-tetrahydro-furan-3-yloxy)-2-trifluoromethyl-benzonltrile, or a salt thereof.

## Patentansprüche

1. Verbindung der Formel: 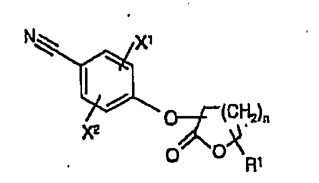 oder ein Salz davon, worin:
a) X¹ Chlor oder Trifluormethyl ist und in Position 2 oder 6 lokalisiert ist;
b) X² fehlt oder für Halogen, Cyano, C₁-C₆-Alkoxy, Halogenalkoxy oder Halogenalkyl steht;
c) n für eine ganze Zahl von 1 bis 4 steht;
d) R¹ für einen Substituenten steht, ausgewählt aus der Gruppe, bestehend aus:
i. Wasserstoff
ii. Halogen,
iii. Cyano,
iv. Hydroxy,
v. (C₁-C₁₂)Alkyl, gegebenenfalls substituiert,
vi. (C₂-C₁₂)Alkenyl, gegebenenfalls substituiert,
vii. (C₂-C₁₂)Alkinyl, gegebenenfalls substituiert,
viii. (C₃-C₁₀)Cycloalkyl, gegebenenfalls substituiert,
ix. (C₃-C₁₀) Cycloalkyl (C₁-C₆) alkyl, worin die Alkyl- und Cycloalkylgruppierungen jeweils gegebenenfalls substituiert sein können,
x. (C₆-C₁₀)Aryl, gegebenenfalls substituiert,
xi. (C₆-C₁₀)Aryl(C₁-C₆)alkyl, worin die Alkyl- und Arylgruppierungen jeweils gegebenenfalls substituiert sein können,
xii. (CH₂)_{z}-SR¹,
xiii. (CH₂)_{z}-O-R¹,
xiv. (CH₂)_{z}-NR¹R²,
xv. (CH₂)_{z}-COOR³,
xvi. (CH₂)_{z}-CONR³R⁴,
xvii. (CH₂)_{z}-NR⁴COR³ und
xviii. (CH₂)_{z}OCOR³,
d) z für eine ganze Zahl von 0 bis 6 steht,
e) R³ für einen Substituenten steht, ausgewählt aus der Gruppe, bestehend aus Wasserstoff, (C₁-C₁₂)Alkyl, (C₂-C₁₂)Alkenyl, (C₂-C₁₂)Alkinyl, gegebenenfalls substituiertes (C₆-C₁₀)Aryl und (C₆-C₁₀)Aryl(C₁-C₆) alkyl, worin die Alkyl- und Arylgruppierungen jeweils gegebenenfalls substituiert sein können, und
f) R⁴ für einen Substituenten steht, ausgewählt aus der Gruppe, bestehend aus Wasserstoff und (C₁-C₁₂)Alkyl.

2. Verbindung nach Anspruch 1, worin n 1 ist.

3. Verbindung nach Anspruch 1 oder 2, worin R¹ ausgewählt ist aus der Gruppe, bestehend aus C₁-C₆-Alkyl, C₁-C₆-Alkoxy, Halogen, Halogenalkyl und Halogenalkoxy.

4. Verbindung nach Anspruch 1, ausgewählt aus der Gruppe, bestehend aus
i) (±)-4-(4,4-Dimethyl-2-oxotetrahydrofuran-3-yloxy)-2-trifluormethylbenzonitril;
ii) (R)-(+)-4-(4,4-Dimethyl-2-oxotetrahydrofuran-3-yloxy)-2-trifluormethylbenzonitril;
iii) (S)-(-)-4-(4,4-Dimethyl-2-oxotetrahydrofuran-3-yloxy)-2-trifluormethylbenzonitril;
iv) (±)-4-(2-Oxotetrahydrofuran-3-yloxy)-2-trifluormethylbenzonitril;
v) (+)-4-(2-Oxotetrahydrofuran-3-yloxy)-2-trifluormethylbenzonitril und
vi) (-)-4-(2-Oxotetrahydrofuran-3-yloxy)-2-trifluormethylbenzonitril.

5. (R)-(+)-4-(4,4-Dimethyl-2-oxotetrahydrofuran-3-yloxy)-2-trifluormethylbenzonitril oder ein pharmazeutisch verträgliches Salz davon.

6. Verbindung nach einem der Ansprüche 1 bis 5 als Medizin.

7. Verwendung einer Verbindung nach einem der Ansprüche 1 bis 5 bei der Herstellung eines Medikaments zur Linderung eines Zustandes, ausgewählt aus der Gruppe, bestehend aus hormonabhängigen Krebsarten, gutartiger Prostatahyperplasie, Akne, Hirsutismus, Sebum-Überschuss, Alopezie, prämenstruellem Syndrom, Lungenkrebs, verfrühter Pubertät, Osteoporose, Hypogonadismus, altersbedingter Abnahme der Muskelmasse und Anämie.

8. Pharmazeutische Zusammensetzung, umfassend eine Verbindung nach einem der Ansprüche 1 bis 5 im Gemisch mit einem oder mehreren pharmazeutisch verträglichen Exzipienzien.

9. Topische pharmazeutische Formulierung, umfassend eine Verbindung nach einem der Ansprüche 1 bis 5 im Gemisch mit einem oder mehreren pharmazeutisch verträglichen Exzipienzien, geeignet zur dermalen Anwendung.

10. Erzeugnis, umfassend eine Verbindung nach einem der Ansprüche 1 bis 5, verpackt zum Einzelhandelsvertrieb, welches einen Konsumenten anweist, wie die Verbindung zu verwenden ist, um einen Zustand, ausgewählt aus der Gruppe, bestehend aus Akne, Alopezie und fettiger Haut, zu lindern.

11. Verwendung einer Verbindung nach einem der Ansprüche 1 bis 5 bei der Herstellung eines Medikaments zur Linderung eines Zustandes, ausgewählt aus der Gruppe, bestehend aus Alopezie, Sebum-Überschuss und Hirsutismus.

12. (±)-4-(2-Oxotetrahydrofuran-3-yloxy)-2-trifluormethylbenzonitril oder ein Salz davon.

## Revendications

1. Composé de formule : 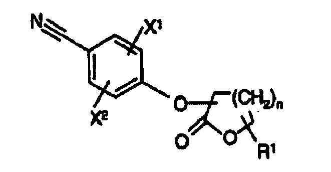 ou sel correspondant, dans lequel :
a) X¹ représente chloro ou trifluorométhyle et est situé en position 2 ou 6 ;
b) X² est absent ou représente halogéno, cyano, alcoxy en C₁-C₆, halogénoalcoxy ou halogénoalkyle ;
c) n représente un entier compris entre 1 et 4 ;
d) R¹ représente un substituant sélectionné dans le groupe consistant en :
i. l'hydrogène,
ii. halogéno,
iii. cyano
iv. hydroxy,
v. alkyle en C₁-C₁₂ facultativement substitué,
vi. alkényle en C₂-C₁₂ facultativement substitué,
vii. alkynyle en C₂-C₁₂ facultativement substitué,
viii. cycloalkyle en C₃-C₁₀ facultativement substitué,
ix. (cycloalkyle en C₃-C₁₀) (alkyle en C₁-C₆), les groupes alkyle et cycloalkyle pouvant chacun être facultativement substitué,
x. aryle en C₆-C₁₀ facultativement substitué,
xi. (aryle en C₆-C₁₀) (alkyle en C₁-C₆), les groupes alkyle et aryle pouvant chacun être facultativement substitué,
xii. (CH₂)_{z}-SR¹,
xiii. (CH₂)_{z}-O-R¹,
xiv. (CH₂)_{z}-NR¹R²,
xv. (CH₂)_{z}-COOR³,
xvi. (CH₂)_{z}-CONR³R⁴,
xvii. (CH₂)_{z}-NR⁴COR³ et
xviii. (CH₂)_{z}-OCOR³,
e) z représente un entier compris entre 0 et 6,
f) R³ représente un substituant sélectionné dans le groupe consistant en l'hydrogène, alkyle en C₁-C₁₂, alkényle en C₂-C₁₂, alkynyle en C₂-C₁₂, aryle en C₆-C₁₀ facultativement substitué et (aryle en C₆-C₁₀) (alkyle en C₁-C₆), les groupes fonctionnels alkyle et aryle pouvant chacun être facultativement substitué ; et
g) R⁴ représente un substituant sélectionné dans le groupe consistant en l'hydrogène ou alkyle en C₁-C₁₂.

2. Composé selon la revendication 1, dans lequel n représente 1.

3. Composé selon la revendication 1 ou 2, dans lequel R¹ est sélectionné dans le groupe consistant en alkyle en C₁-C₆, alcoxy en C₁-C₆, halogéno, halogénoalkyle et halogénoalcoxy.

4. Composé selon la revendication 1, sélectionné dans le groupe consistant en
i) le (±)-4-(4,4-diméthyl-2-oxo-tétrahydro-furan-3-yloxy)-2-trifluorométhyl-benzonitrile ;
ii) le (R)-(+)-4-(4,4-diméthyl-2-oxo-tétrahydro-furan-3-yloxy)-2-trifluorométhyl-benzonitrile ;
iii) le (S)-(-)-4-(4,4-diméthyl-2-oxo-tétrahydro-furan-3-yloxy)-2-trifluorométhyl-benzonitrile ;
iv) le (±)-4-(2-oxo-tétrahydro-furan-3-yloxy)-2-trifluorométhyl-benzonitrile ;
v) le (+)-4-(2-oxo-tétrahydro-furan-3-yloxy)-2-trifluorométhyl-benzonitrile ; et
vi) le (-)-4-(2-oxo-tétrahydro-furan-3-yloxy)-2-trifluorométhyl-benzonitrile.

5. (R)-(+)-4-(4,4-diméthyl-2-oxo-tétrahydro-furan-3-yloxy)-2-trifluorométhyl-benzonitrile ou l'un de ses sels pharmaceutiquement acceptable.

6. Composé selon l'une quelconque des revendications 1 à 5 en tant que médicament.

7. Utilisation d'un composé selon l'une quelconque des revendications 1 à 5, dans la fabrication d'un médicament pour soulager un état sélectionné dans le groupe consistant en les cancers hormono-dépendants, l'hyperplasie bénigne de la prostate, l'acné, l'hirsutisme, l'excès de sébum, l'alopécie, le syndrome prémenstruel, le cancer du poumon, la puberté précoce, l'ostéoporose, l'hypogonadisme, la diminution de la masse musculaire liée à l'âge, et l'anémie.

8. Composition pharmaceutique comprenant un composé selon l'une quelconque des revendications 1 à 5, mélangé à un ou plusieurs excipients pharmaceutiquement acceptables.

9. Formulation pharmaceutique topique comprenant un composé selon l'une quelconque des revendications 1 à 5, mélangé à un ou plusieurs excipients pharmaceutiquement acceptables et convenant à une application dermique.

10. Article manufacturé comprenant un composé selon l'une quelconque des revendications 1 à 5, conditionné pour la distribution au détail, qui informe le consommateur sur la manière d'utiliser le composé pour soulager un état sélectionné dans le groupe consistant en l'acné, l'alopécie et la peau grasse.

11. Utilisation d'un composé selon l'une quelconque des revendications 1 à 5, dans la fabrication d'un médicament pour soulager un état sélectionné dans le groupe consistant en l'alopécie, l'excès de sébum et l'hirsutisme.

12. (±)-4-(2-oxo-tétrahydro-furan-3-yloxy)-2-trifluorométhyl-benzonitrile ou sel correspondant.
